Europäisches Patentamt

European Patent Office

Office européen des brevets

(19)

(11) **EP 0 564 036 B1**

## (12) FASCICULE DE BREVET EUROPEEN

(45) Date de publication et mention
de la délivrance du brevet:
**12.06.1996 Bulletin 1996/24**

(51) Int. Cl.⁶: **C07C 17/38**, C07C 19/08

(21) Numéro de dépôt: **93200877.4**

(22) Date de dépôt: **26.03.1993**

(54) **Procédé d'épuration d'un hydrofluoroalcane**

Verfahren zur Reinigung von einem Hydrofluoralkan

Process for the purification of one hydrofluoralcane

(84) Etats contractants désignés:
**AT BE CH DE DK ES FR GB IT LI NL SE**

(30) Priorité: **03.04.1992 FR 9204277**

(43) Date de publication de la demande:
**06.10.1993 Bulletin 1993/40**

(73) Titulaire: **SOLVAY (Société Anonyme)**
**B-1050 Bruxelles (BE)**

(72) Inventeur: **Darago, Gilles**
**F-39500 Tavaux (FR)**

(74) Mandataire: **Jacques, Philippe et al**
**Solvay S.A.**
**Département Propriété Industrielle**
**310, rue de Ransbeek**
**B-1120 Bruxelles (BE)**

(56) Documents cités:
**EP-A- 0 361 578**      **EP-A- 0 395 793**
**EP-A- 0 395 898**      **EP-A- 0 472 391**
**WO-A-91/06521**      **WO-A-91/18852**

• **CHEMICAL ABSTRACTS, vol. 111, no. 26, 25**
**Décembre 1989, Columbus, Ohio, US; abstract**
**no. 235606b, & JP-A-01 132 539**

EP 0 564 036 B1

## Description

La présente invention se rapporte à un procédé amélioré d'épuration d'un hydrofluoroalcane.

Quel que soit son mode de préparation, un hydrofluoroalcane recueilli à la sortie du réacteur de synthèse, est généralement contaminé par des impuretés comprenant notamment des réactifs non convertis et/ou divers sous-produits générés lors de la réaction ou amenés avec les réactifs. L'hydrofluoroalcane doit donc subir une épuration pour en séparer, d'une part des composés plus volatils que l'hydrofluoroalcane, classiquement appelés impuretés légères et, d'autre part, des composés moins volatils que l'hydrofluoroalcane, classiquement appelés impuretés lourdes.

Un procédé classique bien connu d'épuration des hydrofluoroalcanes consiste en une séparation par distillation en plusieurs étapes. En tête d'une première colonne, habituellement dénommée colonne à légers, sont éliminés du mélange réactionnel les composés plus volatils que l'hydrofluoroalcane, ce dernier quittant cette colonne en pied, en mélange avec les impuretés lourdes. L'épuration de l'hydrofluoroalcane en impuretés lourdes est ensuite menée dans une deuxième colonne de distillation, appelée colonne à lourds, alimentée par le flux soutiré en pied de la colonne à légers, dans laquelle l'hydrofluoroalcane épuré constitue la fraction de tête et les impuretés lourdes constituent la fraction de pied.

Ce procédé classique d'épuration par distillation s'est cependant révélé insuffisant pour obtenir un hydrofluoroalcane répondant aux spécifications de pureté actuellement exigées. Les hydrofluoroalcanes épurés par ce procédé classique sont en effet habituellement contaminés par quelques ppm d'hydracides et d'impuretés organiques, principalement d'impuretés organiques halogénées insaturées, particulièrement gênantes dans le produit final en raison de leur possible toxicité. L'élimination de telles impuretés requiert dès lors généralement un traitement ultérieur de finition.

L'invention vise à remédier aux insuffisances du procédé classique défini ci-dessus, en fournissant un procédé amélioré d'épuration d'un hydrofluoroalcane par distillation, qui permette d'obtenir directement un hydrofluoroalcane quasi exempt d'impuretés organiques halogénées et d'hydracides.

On a maintenant observé que, dans le procédé classique défini plus haut, pendant l'étape d'épuration de l'hydrofluoroalcane en impuretés lourdes par distillation, une fraction de l'hydrofluoroalcane et/ou une fraction de certaines impuretés lourdes, principalement des composés organiques halogénés, peuvent subir une dégradation entraînant l'apparition de composés organiques halogénés insaturés et d'hydracides. Une cause possible de cette dégradation est la présence de traces d'acides de Lewis dans le mélange à séparer. On a en outre observé que, même à température ambiante, la présence de seulement 1 ppm de $FeCl_3$ dans du 1,1-dichloro-1-fluoroéthane suffit pour induire la déshydrochloration d'une fraction significative de ce produit en 1-chloro-1-fluoroéthylène et en chlorure d'hydrogène. Une autre cause possible de cette dégradation est liée à l'utilisation de colonnes de distillation en matériaux métalliques, certains métaux pouvant induire une certaine dégradation des composés halogénés. En raison de leur point d'ébullition inférieur à celui de leurs précurseurs, ces composés de dégradation contaminent, dans le procédé classique d'épuration par distillation, l'hydrofluoroalcane soutiré en tête de la colonne à lourds.

Un autre but de l'invention est dès lors de fournir un procédé d'épuration d'un hydrofluoroalcane en impuretés lourdes par distillation, dans lequel la dégradation de l'hydrofluoroalcane et/ou des impuretés lourdes dans la colonne de distillation est fortement réduite.

La présente invention concerne dès lors un procédé d'épuration d'un hydrofluoroalcane selon lequel on soumet l'hydrofluoroalcane à une distillation pour en séparer des impuretés moins volatiles que ledit hydrofluoroalcane, caractérisé en ce qu'on effectue la distillation en présence d'un capteur d'acidité.

On a observé que, lorsque la distillation est effectuée conformément à l'invention, en présence d'un capteur d'acidité, ni l'hydrofluoroalcane ni les impuretés organiques qui l'accompagnent (en particulier des impuretés organiques halogénées) ne se dégradent de façon perceptible au cours de la distillation, même lorsque celle-ci est réalisée en présence de faibles quantités d'acides de Lewis.

Par hydrofluoroalcanes, on entend généralement désigner les hydrocarbures saturés, de type acyclique ou alicyclique, comprenant au moins un atome d'hydrogène et au moins un atome de fluor. Ils peuvent éventuellement comprendre en outre au moins un atome de chlore. Les hydrofluoroalcanes tels que définis ci-dessus comprennent généralement de 1 à 6 atomes de carbone. Ils répondent en général à la formule $C_aH_bF_cCl_d$ dans laquelle a est un nombre entier de 1 à 6, b est un nombre entier de 1 à 13, c est un nombre entier de 1 à 13 et d est un nombre entier de 0 à 8, avec b + c + d = 2a + 2 lorsque le composé est acyclique et avec b + c + d = 2a lorsque le composé est alicyclique. Le procédé selon l'invention est particulièrement applicable aux composés acycliques comprenant de 1 à 4 atomes de carbone, comprenant au moins un atome d'hydrogène et au moins un atome de fluor. Ces composés peuvent en outre contenir au moins un atome de chlore. Ils répondent à la formule ci-dessus dans laquelle a est un nombre entier de 1 à 4, b est un nombre entier de 1 à 9, c est un nombre entier de 1 à 9 et d est un nombre entier de 0 à 5. Le procédé est tout particulièrement applicable aux composés acycliques répondant à la formule générale ci-dessus dans laquelle a est un nombre entier égal à 2 ou 3, b est un nombre entier de 1 à 6, c est un nombre entier de 1 à 6 et d est un nombre entier de 1 à 4. A titre d'exemples d'hydrofluoroalcanes pouvant être traités par le procédé de l'invention, on peut mentionner les composés de formule $CH_3CCl_2F$, $CH_3CClF_2$, $CH_3CHF_2$, $CH_3CF_3$, $CH_2FCH_2F$, $CH_2FCHF_2$, $CH_2FCF_3$, $CHF_2CCl_3$, $CHF_2CF_3$, $CHCl_2CF_3$,

CHClFCF$_3$, CHF$_2$CHF$_2$, CF$_3$CF$_2$CHF$_2$, CF$_3$CF$_2$CHCl$_2$, CF$_2$ClCF$_2$CHClF, CF$_3$CF$_2$CH$_3$, CF$_3$CH$_2$CF$_2$CH$_3$ et CF$_3$CH$_2$CH$_2$CF$_3$. De bons résultats ont été obtenus avec le procédé selon l'invention lorsqu'il est appliqué à l'épuration du 1,1-dichloro-1-fluoroéthane.

Les hydrofluoroalcanes tels que définis ci-dessus, auxquels le procédé selon l'invention est applicable, sont généralement obtenus par réaction d'un halogène, d'un hydracide ou d'hydrogène avec un hydrocarbure saturé ou insaturé, éventuellement halogéné. A titre d'exemples de telles réactions, on peut citer la synthèse du 1,1-dichloro-1-fluoroéthane par hydrofluoration du chlorure de vinylidène ou du 1,1,1-trichloroéthane. On peut aussi mentionner la synthèse du 1,1,1,2-tétrafluoroéthane par hydrofluoration catalytique d'un composé de formule CX$_3$CH$_2$Cl, avec X égal à Cl ou F. Les conditions opératoires dans lesquelles ces réactions sont mises en oeuvre sont bien connues dans l'art antérieur. Quel que soit l'hydrofluoroalcane synthétisé et le mode de préparation utilisé, l'hydrofluoroalcane sortant du réacteur de synthèse est généralement contaminé par des impuretés diverses. Ces impuretés peuvent avoir de multiples origines et leur nature dépend de l'hydrofluoroalcane synthétisé et du mode de préparation utilise. Il peut s'agir notamment de réactifs non convertis lors de la synthèse de l'hydrofluoroalcane, d'impuretés ou de dérivés d'impuretés présentes dans les réactifs, de sous-produits formés au cours de la synthèse ou encore de réactifs mis en oeuvre ou de produits obtenus dans un traitement postérieur à la synthèse pour transformer chimiquement certaines impuretés accompagnant l'hydrofluoroalcane.

Dans le procédé selon l'invention, les impuretés moins volatiles que l'hydrofluoroalcane sont des composés chimiques, généralement organiques, dont le point d'ébullition est supérieur au point d'ébullition de l'hydrofluoroalcane soumis à l'épuration. En règle générale, il est souhaitable, pour des considérations économiques, que la volatilité relative, c'est-à-dire le rapport entre la volatilité de l'hydrofluoroalcane et la volatilité des impuretés moins volatiles que l'hydrofluoroalcane, soit d'au moins 1,05, de préférence d'au moins 1,1.

Par la suite, les impuretés moins volatiles que l'hydrofluoroalcane, telles que décrites ci-dessus, seront désignées impuretés lourdes. Les impuretés lourdes sont habituellement des composés organiques halogénés en C$_2$-C$_8$, principalement des hydrocarbures aliphatiques saturés, notamment d'autres hydrofluoroalcanes que celui soumis à l'épuration.

Pour inhiber efficacement la dégradation de l'hydrofluoroalcane à épurer et des impuretés lourdes pendant la distillation, le capteur d'acidité utilisé dans le procédé selon l'invention doit présenter une grande réactivité à l'égard des hydracides et des halogènes et posséder une tension de vapeur suffisante pour être réparti de manière efficace dans la colonne où on effectue la distillation. Des capteurs d'acidité utilisables dans le procédé selon l'invention sont notamment des époxydes, des amines et des hydrocarbures insaturés. Comme époxydes, on peut mentionner notamment le 1,2-époxypropane, le 1,2-époxybutane, le 1,2-époxypentane, le 1,2-époxycyclohexane, le 1,2-époxyéthylbenzène, l'épichlorhydrine et le glycidol. Comme amines, on peut mentionner la diéthylamine, la dipropylamine, la diisopropylamine, la dibutylamine, la diisobutylamine, la triéthylamine et la tripropylamine. Comme hydrocarbures insaturés on peut mentionner notamment les propènes, les butènes, les méthylbutènes, les diméthylbutènes, les pentènes, les méthylpentènes, les diméthylpentènes, les triméthylpentènes, les hexènes, le cyclopentène, le cyclohexène et le méthylcyclopentène. Selon l'invention, les capteurs d'acidité préférés sont les hydrocarbures insaturés en C$_3$-C$_8$. Parmi ces hydrocarbures insaturés, le 2-méthylbut-2-ène, le 2-méthylbut-1-ène, le 3-méthylbut-1-ène, le 2-éthylbut-1-ène, le pent-1-ène, le pent-2-ène, le 2-méthylpent-1-ène, le 2-méthylpent-2-ène, le 3-méthylpent-1-ène, le 3-méthylpent-2-ène, le 4-méthylpent-1-ène, le 2,4,4-triméthylpent-1-ène, le hex-1-ène, le hex-2-ène et le hex-3-ène sont particulièrement préférés.

Dans le cas où le procédé est appliqué à l'épuration du 1,1-dichloro-1-fluoroéthane, l'hydrocarbure insaturé tout particulièrement préféré comme capteur d'acidité est le 2-méthylbut-2-ène. Dans ce cas, de très bons résultats ont été obtenus avec du 2-méthylbut-2-ène industriel, habituellement dénommé amylène, lequel renferme en outre un peu de 2-méthylbut-1-ène.

Toutes autres choses étant égales par ailleurs, la quantité optimum de capteur d'acidité à mettre en oeuvre va dépendre de la nature de l'hydrofluoroalcane à épurer, de la nature des impuretés lourdes et de leur concentration par rapport à l'hydrofluoroalcane à épurer, ainsi que du capteur d'acidité sélectionné. Elle doit dès lors être déterminée dans chaque cas particulier par un travail de routine en laboratoire. En pratique, on recommande de mettre en oeuvre une quantité pondérale de capteur d'acidité au moins égale à 0,005 g par kilo d'hydrofluoroalcane soumis à la distillation, de préférence supérieure ou égale à environ 0,01 g. On n'a généralement pas intérêt à ce que cette quantité excède 1 g par kilo d'hydrofluoroalcane soumis à la distillation, de préférence environ 0,1 g. Les quantités situées entre environ 0,025 g et environ 0,075 g par kilo d'hydrofluoroalcane soumis à la distillation sont recommandées.

Dans le procédé selon l'invention, la distillation est généralement effectuée dans une ou plusieurs colonnes à distiller, qui sont bien connues dans le domaine de l'art. Dans cette forme de réalisation du procédé selon l'invention, les meilleurs résultats sont obtenus lorsque le capteur d'acidité est réparti de manière homogène dans toute la colonne à distiller. Le capteur d'acidité peut être introduit à différents niveaux de la colonne. Pour assurer une efficacité maximale, il est parfois désirable d'introduire le capteur d'acidité en plusieurs endroits de la colonne. Le capteur d'acidité peut être introduit périodiquement dans la colonne ou de préférence, de façon continue.

Dans une première variante de la forme de réalisation précitée du procédé, on introduit le capteur d'acidité en tête de la colonne à distiller, par exemple en l'injectant dans une fraction de l'hydrofluoroalcane condensé qui est renvoyée dans la colonne comme reflux.

Dans une deuxième variante de cette forme de réalisation du procédé, on introduit le capteur d'acidité en pied de la colonne à distiller, par exemple en l'injectant dans un bouilleur de la colonne.

Dans une troisième variante de cette forme de réalisation du procédé, on ajoute le capteur d'acidité à l'hydrofluoroalcane en amont de la colonne à distiller.

Dans la première variante précitée du procédé, on peut mettre en oeuvre un capteur d'acidité présentant un point d'ébullition nettement plus élevé que celui de l'hydrofluoroalcane à épurer, par exemple un composé dont le point d'ébullition est supérieur de 15 °C à 50 °C par rapport au point d'ébullition de l'hydrofluoroalcane à épurer. Cette variante du procédé est préférée lorsque la teneur en capteur d'acidité dans l'hydrofluoroalcane recueilli de l'épuration doit rester très faible, par exemple inférieure à 5 ppm.

Lorsque l'on cherche à obtenir une teneur résiduelle en capteur d'acidité dans l'hydrofluoroalcane recueilli de l'épuration supérieure à 5 ppm, par exemple de l'ordre de 10 à 100 ppm, on choisit, conformément à l'invention, un capteur d'acidité dont le point d'ébullition est proche de celui de l'hydrofluoroalcane, par exemple, un composé dont le point d'ébullition ne diffère pas de celui de l'hydrofluoroalcane à épurer de plus de 15 °C, de préférence, un composé dont le point d'ébullition ne diffère pas de celui de l'hydrofluoroalcane à épurer de plus de 10 °C.

De manière particulièrement préférée, on utilisera plutôt un capteur d'acidité dont le point d'ébullition est de 1 à 5 °C supérieur à celui de l'hydrofluoroalcane à épurer. Un tel capteur d'acidité peut être mis en oeuvre dans les trois variantes du procédé décrites plus haut. La troisième variante du procédé apparaît particulièrement avantageuse et est dès lors préférée.

Dans le procédé selon l'invention, il est nécessaire que l'hydrofluoroalcane soumis à la distillation soit sensiblement exempt d'impuretés plus volatiles que lui.

Les impuretés plus volatiles que l'hydrofluoroalcane sont des composés chimiques inorganiques ou organiques, dont le point d'ébullition est inférieur au point d'ébullition de l'hydrofluoroalcane soumis à l'épuration.

Par la suite, les impuretés plus volatiles que l'hydrofluoroalcane, telles que définies ci-dessus, seront désignées impuretés légères. Typiquement, des impuretés légères de l'hydrofluoroalcane comprennent des gaz inertes comme de l'azote, des hydracides comme le fluorure d'hydrogène et le chlorure d'hydrogène, des halogènes comme le chlore et le fluor, de l'hydrogène ainsi que certains composés organiques, principalement des composés organiques en $C_1$-$C_4$, le plus souvent halogénés, éventuellement insaturés.

Les impuretés légères, sont éliminées de l'hydrofluoroalcane par toute technique appropriée, avant l'introduction du capteur d'acidité dans le mélange.

Dans une forme préférée d'exécution du procédé selon l'invention, avant la distillation, on soumet l'hydrofluoroalcane à une prédistillation pour en séparer des impuretés légères. La prédistillation est une distillation qui précède celle destinée à séparer les impuretés lourdes de l'hydrofluoroalcane et qui est opérée dans les conditions réglées pour séparer les impuretés légères de l'hydrofluoroalcane. La prédistillation est effectuée dans une ou plusieurs colonnes à distiller.

Il est généralement souhaitable d'ajouter le capteur d'acidité le plus rapidement possible après la séparation des impuretés légères de l'hydrofluoroalcane. A cet effet, dans la forme d'exécution préférée décrite ci-dessus où les impuretés légères sont éliminées par une prédistillation, on peut injecter le capteur d'acidité dans le bouilleur de la colonne où on effectue la prédistillation, endroit où l'hydrofluoroalcane n'est plus contaminé par des quantités significatives d'impuretés légères, particulièrement d'halogènes ou d'hydracides.

Comme décrit plus haut, selon les variantes d'exécution du procédé et la nature du capteur d'acidité mis en oeuvre, le procédé selon l'invention permet d'obtenir l'hydrofluoroalcane épuré, soit quasi exempt de capteur d'acidité, soit renfermant de faibles quantités pondérales de capteur d'acidité, par exemple de l'ordre de 10 à 100 ppm. A ce niveau de concentration, le capteur d'acidité joue alors le rôle de stabilisant de l'hydrofluoroalcane, tant au stockage que dans ses utilisations ultérieures, notamment comme solvant.

L'invention présente de nombreux avantages. Elle permet notamment d'effectuer la distillation dans une colonne en acier ordinaire. Elle permet aussi d'effectuer la distillation en présence d'acides de Lewis, régulièrement présents dans les hydrofluoroalcanes à épurer en raison de leur fréquente utilisation comme catalyseurs, au cours de la synthèse des hydrofluoroalcanes. L'inhibition de la formation, au sein de la colonne à distiller, de produits organiques insaturés et d'hydracides, plus légers que l'hydrofluoroalcane soumis à l'épuration, permet de récupérer ce dernier en tête de la colonne, opération plus aisée qu'un soutirage sur un plateau intermédiaire. L'absence de composés plus volatils que l'hydrofluoroalcane dans la colonne rend en outre inutile une purge en tête, ce qui améliore le rendement global du procédé. L'invention permet en outre de mener la distillation à des pressions et températures plus élevées qu'en absence de capteur d'acidité sans que l'on observe de dégradation de l'hydrofluoroalcane ou des impuretés lourdes. Elle permet aussi d'obtenir un hydrofluoroalcane répondant aux spécifications de pureté les plus strictes sans devoir mettre en oeuvre un traitement de finition du produit.

Les exemples dont la description suit servent à illustrer l'invention. L'exemple 1, réalisé selon un procédé classique d'épuration par distillation, est donné à titre de référence. Les exemples 2 et 3 sont conformes à l'invention.

Exemple 1 (de référence)

Du 1,1-dichloro-1-fluoroéthane obtenu par hydrofluoration de chlorure de vinylidène, puis ayant subi un traitement de chloration des impuretés insaturées en présence de $FeCl_3$, est soumis à une prédistillation pour l'épurer en impuretés légères. A cet effet, il est introduit dans une première colonne de rectification (colonne à légers), sous une pression de 2,1 bar. Le chlore excédentaire mis en oeuvre dans l'étape précédente de chloration est éliminé en tête de colonne et le mélange de 1,1-dichloro-1-fluoroéthane et des impuretés lourdes comprenant notamment les composés saturés chlorés produits dans l'étape de chloration des impuretés insaturées quitte la colonne à légers en pied. Ce mélange est ensuite introduit dans une seconde colonne à distiller (colonne à lourds), fonctionnant à une pression de 1,5 bar. Dans cette colonne, le mélange subit une distillation pour séparer le 1,1-dichloro-1-fluoroéthane des impuretés qui l'accompagnent. Le 1,1-dichloro-1-fluoroéthane y est soutiré en tête de colonne et les impuretés lourdes sont éliminées en pied. Le taux de reflux est égal à 2.

La concentration moyenne en 1-chloro-1-fluoroéthylène dans le 1,1-dichloro-1-fluoroéthane soutiré en tête est de 5 mg par kilo de 1,1-dichloro-1-fluoroéthane.

Exemple 2 (selon l'invention)

L'exemple 1 ci-dessus est répété en tous points, mais on injecte 30 mg d'amylène par kilo de mélange dans le bouilleur de la colonne à légers. La concentration moyenne en 1-chloro-1-fluoroéthylène dans le 1,1-dichloro-1-fluoroéthane soutiré en tête est de moins de 0,2 mg par kilo de 1,1-dichloro-1-fluoroéthane.

Le 1,1-dichloro-1-fluoroéthane épuré renferme en moyenne 20 mg d'amylène par kilo.

Exemple 3 (selon l'invention)

L'exemple 1 ci-dessus est répété en tous points, mais on injecte 30 mg d'amylène par kilo de 1,1-dichloro-1-fluoroéthane renvoyé à la colonne à lourds comme reflux.

La concentration moyenne en 1-chloro-1-fluoroéthylène dans le 1,1-dichloro-1-fluoroéthane soutiré en tête est de moins de 0,2 mg par kilo de 1,1-dichloro-1-fluoroéthane et le 1,1-dichloro-1-fluoroéthane épuré renferme en moyenne 50 mg d'amylène par kilo.

**Revendications**

1. Procédé d'épuration d'un hydrofluoroalcane selon lequel on soumet l'hydrofluoroalcane à une distillation pour en séparer des impuretés moins volatiles que ledit hydrofluoroalcane, caractérisé en ce qu'on effectue la distillation en présence d'un capteur d'acidité.

2. Procédé selon la revendication 1, caractérisé en ce que l'on met en oeuvre un hydrofluoroalcane qui a subi une prédistillation pour en séparer des impuretés plus volatiles que ledit hydrofluoroalcane.

3. Procédé selon la revendication 1 ou 2, caractérisé en ce que le capteur d'acidité est un hydrocarbure insaturé en $C_3$-$C_8$.

4. Procédé selon une quelconque des revendications 1 à 3, caractérisé en ce que la quantité de capteur d'acidité est de 0,005 g à 1 g par kilo d'hydrofluoroalcane soumis à la distillation.

5. Procédé selon la revendication 4, caractérisé en ce que la quantité de capteur d'acidité est de 0,01 g à 0,1 g par kilo d'hydrofluoroalcane soumis à la distillation.

6. Procédé selon une quelconque des revendications 1 à 5, caractérisé en ce que le capteur d'acidité présente un point d'ébullition supérieur de 15 °C à 50 °C au point d'ébullition de l'hydrofluoroalcane à épurer et en ce qu'on l'introduit en tête d'une colonne dans laquelle on effectue la distillation.

7. Procédé selon une quelconque des revendications 1 à 5, caractérisé en ce qu'on met en oeuvre un capteur d'acidité dont le point d'ébullition ne diffère pas de celui de l'hydrofluoroalcane à épurer de plus de 15 °C.

8. Procédé selon la revendication 7, caractérisé en ce qu'on met en oeuvre un capteur d'acidité dont le point d'ébullition est de 1 à 5 °C supérieur à celui de l'hydrofluoroalcane à épurer.

9. Procédé selon la revendication 7 ou 8, caractérisé en ce qu'on ajoute le capteur d'acidité à l'hydrofluoroalcane en amont de la colonne où on effectue la distillation.

10. Procédé selon une quelconque des revendications 1 à 9 caractérisé en ce qu'il est appliqué à l'épuration de 1,1-dichloro-1-fluoréthane.

11. Procédé selon la revendication 10 caractérisé en ce qu'on met en oeuvre du 2-méthylbut-2-ène comme capteur d'acidité.

12. Procédé selon l'une quelconque des revendications 7 à 11, pour l'obtention d'un hydrofluoroalcane renfermant de 10 à 100 mg de capteur d'acidité par kilo.

**Claims**

1. Purification process for a hydrofluoroalkane, according to which the hydrofluoroalkane is subjected to a distillation in order to separate therefrom the impurities which are less volatile than the said hydrofluoroalkane, characterised in that the distillation is performed in the presence of an acid-scavenger.

2. Process according to Claim 1, characterised in that a hydrofluoroalkane is used which has undergone a predistillation in order to separate therefrom impurities which are more volatile than the said hydrofluoroalkane.

3. Process according to Claim 1 or 2, characterised in that the acid-scavenger is a $C_3$-$C_8$ unsaturated hydrocarbon.

4. Process according to any one of Claims 1 to 3, characterised in that the quantity of acid-scavenger is of 0.005 g to 1 g per kilo of hydrofluoroalkane subjected to the distillation.

5. Process according to Claim 4, characterised in that the quantity of acid-scavenger is of 0.01 g to 0.1 g per kilo of hydrofluoroalkane subjected to the distillation.

6. Process according to any one of Claims 1 to 5, characterised in that the acid-scavenger has a boiling point higher by 15°C to 50°C than the boiling point of the hydrofluoroalkane to be purified, and in that it is introduced at the top of the column in which the distillation is performed.

7. Process according to any one of Claims 1 to 5, characterised in that an acid-scavenger is used whose boiling point differs from that of the hydrofluoroalkane to be purified by no more than 15°C.

8. Process according to Claim 7, characterised in that an acid-scavenger is used whose boiling point is from 1 to 5°C higher than that of the hydrofluoroalkane to be purified.

9. Process according to Claim 7 or 8, characterised in that the acid-scavenger is added to the hydrofluoroalkane upstream of the column in which the distillation is performed.

10. Process according to any one of Claims 1 to 9, characterised in that it is applied to the purification of 1,1-dichloro-1-fluoroethane.

11. Process according to Claim 10, characterised in that 2-methylbut-2-ene is used as the acid-scavenger.

12. Process according to any one of Claims 7 to 11 for obtaining a hydrofluoroalkane containing from 10 to 100 mg of acid-scavenger per kilo.

**Patentansprüche**

1. Verfahren zur Reinigung eines Hydrofluoralkans, gemäß dem man das Hydrofluoralkan destilliert, um Verunreinigungen, die weniger flüchtig als das besagte Hydrofluoralkan sind, daraus abzutrennen, dadurch gekennzeichnet, daß man die Destillation in Gegenwart eines Säurefängers ausführt.

2. Verfahren gemäß Anspruch 1, dadurch gekennzeichnet, daß man ein Hydrofluoralkan einsetzt, das vordestilliert wurde, um Verunreinigungen, die flüchtiger als besagtes Hydrofluoralkan sind, daraus abzutrennen.

3. Verfahren gemäß Anspruch 1 oder 2, dadurch gekennzeichnet, daß der Säurefänger ein ungesättigter $C_3$ - $C_8$ - Kohlenwasserstoff ist.

4. Verfahren gemäß einem der Ansprüche 1 bis 3, dadurch gekennzeichnet daß die Menge an Säurefänger 0,005 g bis 1 g pro Kilogramm Hydrofluoralkan, das destilliert wird, beträgt.

5. Verfahren gemäß Anspruch 4, dadurch gekennzeichnet, daß die Menge an Säurefänger 0,01 g bis 0,1 g pro Kilogramm Hydrofluoralkan, das destilliert wird, beträgt.

6. Verfahren gemäß einem der Ansprüche 1 bis 5, dadurch gekennzeichnet, daß der Säurefänger einen Siedepunkt aufweist, der 15 °C bis 50 °C höher als der Siedepunkt des zu reinigenden Hydrofluoralkans ist, und daß man ihn am Kopf einer Kolonne zugibt, in der man die Destillation durchführt.

7. Verfahren gemäß einem der Ansprüche 1 bis 5, dadurch gekennzeichnet daß man einen Säurefänger einsetzt, dessen Siedepunkt nicht mehr als 15 °C von dem des zu reinigenden Hydrofluoralkans abweicht.

8. Verfahren gemäß Anspruch 7, dadurch gekennzeichnet, daß man einen Säurefänger einsetzt, dessen Siedepunkt 1 bis 5 °C höher als der des zu reinigenden Hydrofluoralkans ist.

9. Verfahren gemäß Anspruch 7 oder 8, dadurch gekennzeichnet, daß man den Säurefänger vor der Kolonne, in der man die Destillation durchführt, zu dem Hydrofluoralkan zugibt.

10. Verfahren gemäß einem der Ansprüche 1 bis 9, dadurch gekennzeichnet, daß es für die Reinigung von 1,1-Dichlor-1-fluorethan angewendet wird.

11. Verfahren gemäß Anspruch 10, dadurch gekennzeichnet, daß man 2-Methylbut-2-en als Säurefänger einsetzt.

12. Verfahren gemäß einem der Ansprüche 7 bis 11 für den Erhalt eines Hydrofluoralkans, das 10 bis 100 mg Säurefänger pro Kilogramm enthält.